# EUROPEAN PATENT SPECIFICATION

(11) **EP 1 585 552 B1**
(45) Date of publication and mention of the grant of the patent: **20.02.2008**
(21) Application number: 03815459.7
(22) Date of filing: 12.06.2003
(51) Int. Cl.: A61L 9/01, B01J 20/02, B01J 23/04, B01J 23/70, A61L 9/012

(54) **MULTI-LAYERED, HOLLOW AND BALL-SHAPED CARBON NANO-STRUCTURE FOR DEODORIZATION**
MEHRSCHICHTIGE, HOHLE UND NANOSKALIGE KOHLENSTOFFKUGEL ZUR DESODORISIERUNG
NANO-BALLE EN CARBONE MULTICOUCHE ET CREUSE POUR LA DESODORISATION

(30) Priority: 17.01.2003 KR 2003003367
(43) Date of publication of application: 19.10.2005
(73) Proprietor: LG Household & Health Care Ltd., Seoul 150-721 (KR)
(72) Inventor: KIM, Jong-Yun, 150-721 Seoul (KR); SONG, Jun-Yeob,133-502 Hanil-Town Apt., Gyeongg-do 440-709 (KR); PARK, Seung-Kyu, Daejeon 305-728 (KR); KANG, Yun-Seog, Daejeon 305-761 (KR); YOON, Suk-Bon, Daejeon 302-110 (KR); YU, Jong-Sung, Daejeon 302-776 (KR)
(74) Representative: Hinkelmann, Klaus
(86) International application number: PCT/KR2003/001155
(87) International publication number: WO 2004/064877

(56) References cited:
- EP-A- 0 893 128
- EP-A- 1 300 364
- WO-A-01/89991
- WO-A-02/38520
- KR-A- 19990 068 330
- KR-A- 20030 072 077
- US-A- 4 370 301
- LEE J ET AL: "DEVELOPMENT OF A NEW MESOPOROUS CARBON USING AN HMS ALUMINOSILICATETEMPLATE" ADVANCED MATERIALS, WILEY VCH, WEINHEIM, DE, vol. 12, no. 5, 2 March 2000 (2000-03-02), pages 359-362, XP000919780 ISSN: 0935-9648
- PATENT ABSTRACTS OF JAPAN vol. 2002, no. 03, 3 April 2002 (2002-04-03) & JP 2001 321424 A (DENSO CORP), 20 November 2001 (2001-11-20)
- YOON S. B. ET AL: 'FABRICATION OF CARBON CAPSULES WITH HOLLOW MACROPOROUS CORE/MESOPOROUS SHELL STRUCTURES' ADV. MATER. vol. 14, no. 1, 04 January 2002, pages 19 - 21, XP001129559

## Description

### TECHNICAL FIELD

The present invention relates to a ball shaped carbon structure for deodorization, and more particularly to a multi-layered ball shaped carton structure for deodorization, which is composed of a porous carbon shell having a spherical hollow core.

### BACKGROUND ART

Generally, various bad smells are generated from daily necessaries such as refrigerator, air conditioner, diaper, hygienic band, cigarette, footwear cabinet and clothes chest and in daily life area such as bedroom, bathroom and automobile room. In addition, bad smells are also generated from exhaust gas of automobiles and industrial equipments such as refuse disposal plant, wastewater disposal plant and factories. Materials generating bad smells are representatively as follows: methanethiol, methyl sulfide, dimethyl disulfide, hydrogensulfide, ammonia, trimethylamine, acetaldehyde, nitric oxide, nitrous oxide, styrene and so on.

Also, various kinds of deodorizing agents have been developed in order to eliminate such bad smells.

Recently, a method for making a carbon nanoball composed of a porous carbon shell having a spherical hollow core (Adv. Mater. 2002, 14, no. 1, January 4) was proposed. This carbon nanoball has an advantage that it may adsorb more various kinds of malodor substances than a conventional activated carbon deodorizing agent. However, the carbon nanoball has some limitations that it may not adsorb any more malodor substances after adsorbing a certain amount. In addition, the above-mentioned carbon nanoball has a limited capacity in deodorizing.

### DISCLOSURE OF INVENTION

The present invention is designed to solve such drawbacks of the prior art, and therefore an object of the present invention is to provide a carbon nanoball having excellent deodorizing ability and capable of adsorbing various kinds of malodor substances.

In order to accomplish the above object, the present invention provides a ball-shaped carbon structure for deodorization comprising a porous carbon shell having a spherical hollow core, wherein at least one deodorizing material selected from the group consisting of transition metal, oxidized transition metal and alkali metal salt is impregnated to the shell, wherein the porous carbon shell has a multi-layered structure in which at least two layers having different pore sizes are included, and wherein an average diameter of pores formed in an outer layer is larger than an average diameter of pores formed in an inner layer or vice versa and wherein the spherical hollow core (15) has a diameter of 5 ~ 1,000nm, and the porous carbon shell (13) has a thickness of 10 ~ 500nm.

Preferably, the transition metal is one selected from the group consisting of Copper (Cu), Iron (Fe), Manganese (Mn), Nickel (Ni), Cobalt (Co), Silver (Ag), Gold (Au), Vanadium (V), Ruthenium (Ru), Titanium (Ti), Chrome (Cr), Zinc (Zn) and Palladium (Pd), and the alkali metal salt is one selected from the group consisting of sodium bromide (NaBr), sodium iodide (NaI), potassium bromide (KBr), potassium iodide (KI) and potassium iodate (KIO₃).

### BRIEF DESCRIPTION OF THE DRAWINGS

These and other features, aspects, and advantages of preferred embodiments of the present invention will be more fully described in the following detailed description, taken accompanying drawings. In the drawings:
FIG. 1 is a schematic diagram for illustrating the process for making a ball shaped carbon structure for deodorization according to the present invention;
FIG. 2 is a photograph of a multi-layered ball shaped carbon structure according to the present invention, taken by an electronic microscope; and
FIG. 3 is a graph dynamic absorption behavior of the multi-layered ball shaped carbon structure for deodorization according to the present invention.

### BEST MODES FOR CARRYING OUT THE INVENTION

Hereinafter, embodiments of the present invention will be described, however the present invention is not limited to the following embodiments, but capable of being modified in diverse ways within the scope of the invention.

A ball shaped carbon structure for deodorization of the present invention is composed of a hollow core and a porous shell wherein the spherical hollow core (15) has a diameter of 5 ~ 1,000nm, and the porous carbon shell (13) has a thickness of 10 ~ 500nm. Transition metal, oxidized transition metal, alkali metal salt or their mixture is impregnated to the shell. In addition, the porous carbon shell has at least two layers of different pore sizes. Pores formed in the outer layer of the shell can have an average diameter larger than pores formed in the inner layer or vice versa, so various kinds of malodor substances may be absorbed in order depending on their size, shape or chemical characteristics. The size of the pores is controlled in the range of 0.01 to 50nm.

Since the shell has at least two layers of different pore sizes, various kinds of malodor substances may be absorbed, and the deodorizing materials impregnated in the pores and on the inner and outer surfaces of the shell may chemically adsorb and destroy the malodor substances. This ball shaped carbon structure for deodorization of the present invention shows more excellent deodorizing ability than the impregnated activated carbon disclosed in Korean Laid-open Patent Publication No. 1999-80808. In other words, since the activated carbon has microporous pores, the pores may be clogged to deteriorate the deodorizing ability when the deodorizing materials are impregnated. However, since the ball shaped carbon structure for deodorization of the present invention has the mesoporous pores in the shell, such a problem does not occur. In addition, since the ball shaped carbon structure for deodorization of the present invention captures the malodor substances in the hollow core thereof, differently to the impregnated activated carbon, it is possible to give sufficient contact time between the malodor substances and the deodorizing material impregnated on the inner surface of the shell. In addition, the ball shaped carbon structure of the present invention may prevent secondary pollution caused when decomposition products generated by the deodorizing materials are emitted outside.

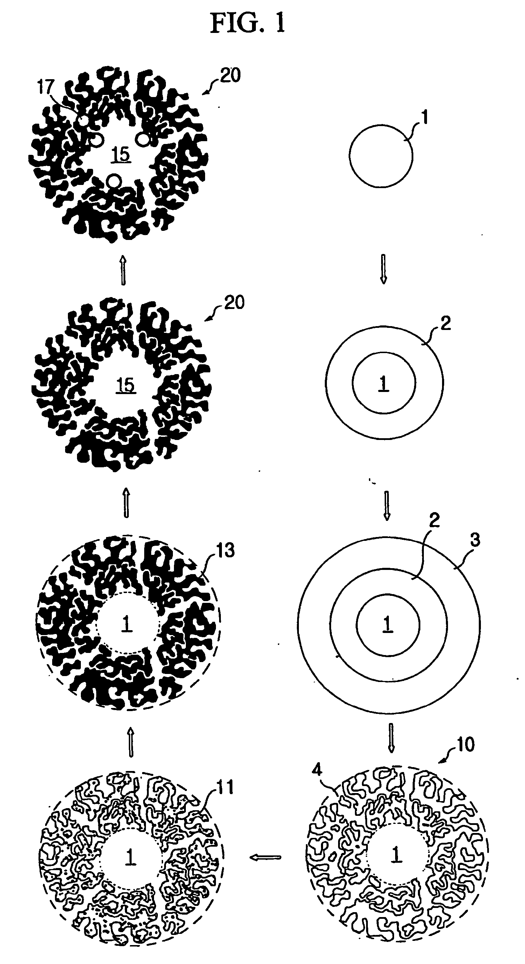

A method for making the ball shaped carbon structure for deodorization according to the present invention is described in detail with reference to FIG. 1.

At first, a spherical silica core 1 is prepared. The silica core 1 may be composed according to the well-known Stober method (Stober, W.; Fink, A.; Bohn, E. J. Colloid Inter. Sci. 1968, 26, 62) from a silica precursor such as tetramethylorthosilicate and tetraethylorthosilicate, as an example. The silica core preferably has a diameter of 5 to 1,000nm.

After that, a first shell 2 made of silica and surface active agent is grown up on the surface of the silica core 1 by reacting silica precursor and surface active agent such as alkyltrimethoxysilane expressed by the following Chemical Formula 1, alkyltriethoxysilane expressed by the following Chemical Formula 2, halogenated alkyltrimethylammonium expressed by the following Chemical Formula 3, alkylpolyoxyethylene expressed by the following Chemical Formula 4 and glycerolethoxylate expressed by the following Chemical Formula 5, in a solvent.

Chemical Formula 1 R₁R₂R₃R₄Si(OCH₃)₃

In the Chemical Formula 1, R₁, R₂ and R₃ are methyl or ethyl groups independently, and R₄ is an alkyl group having a carbon number of 12 to 22.

Chemical Formula 2 R₁R₂R₃R₄Si(OC₂H₅)₃

In the Chemical Formula 2, R₁, R₂ and R₃ are methyl or ethyl groups independently, and R₄ is an alkyl group having a carbon number of 12 to 22.

Chemical Formula 3 R₁R₂R₃R₄NX

In the Chemical Formula 3, R₁, R₂ and R₃ are independently methyl or ethyl groups, R₄ is an alkyl group having a carbon number of 4 to 22, and X is halogen.

Chemical Formula 4 R(OCH₂CH₂)nOH

In the Chemical Formula 4, R is an alkyl group having a carbon number of 4 to 22, and n is an integer in the range of 3 ~ 20.

Chemical Formula 5 CH₂(CH₂O)n₁HCH(CH₂O)n₂HCH₂(CH₂O)n₃H

In the Chemical Formula 5, n₁, n₂ and n₃ are independently integers in the range of 4 ~ 20.

Then, silica precursor and surface active agent having different kind and molar ratio are added and reacted to form a second shell 3 on the surface of the first shell 2. The size of pores formed in the shell is changed depending on the kind of the surface active agent and the kind and molar ratio of the silica precursor. Thus, the kinds of silica precursor and surface active agent are controlled so that the pores formed in the second shell 3 have a larger size than the pores formed in the first shell 2. When required, a third shell having larger pores may be subsequently formed on the surface of the second shell 3.

After that, after the product in which the shell is formed is selectively filtered and then calcined at, for example, 500 ~ 600°C to remove the surface active agent components. Then, a particle 10 having a multi-layered silica shell 4 in which mesoporous pores having a certain size are formed in the place where the surface active agent is removed is obtained. The multi-layered silica shell 4 preferably has a thickness of 10 to 500nm.

Subsequently, a monomer 11 such as acrylonitrile, phenol-formaldehyde and divinylbenzene, sugar, furfuryl, and so on, which are capable of forming polymer as a carbon precursor, is injected into the pores formed in the shell of the particle 10 in which the multi-layered silica shell is formed. After that, the monomer is polymerized to form a polymer. As an example of polymerization, when the radical polymerization is utilized to form a polymer as a carbon precursor, the monomer is sufficiently mixed with a radical initiator and then injected into the mesoporous pores of the silica particle, and then polymerized according to the characteristics of the monomer. At this time, azobisisobutyronitrile (AIBN), t-butyl peracetate, benzoyl peroxide, acetyl peroxide and lauryl peroxide may be used for the radical initiator. This polymerization is well known in the art, and preferably conducted for about 12 hours at 60 to 130°C to make the silica/polymer composite.

And then, the silica/polymer composite is treated under the nitrogen atmosphere at about 1,000°C so as to make the silica containing a carbonized polymer 13. After that, the silica/polymer composite is put into a hydrofluoric acid solution or a sodium hydroxide/ethyl alcohol mixed solution to remove the silica structure, then a ball shaped carbon structure 20 which has a spherical hollow core 15 and a porous carbon shell is obtained.

After that, the ball shaped carbon structure 20 is dipped into a deodorizing material solution composed of transition metal, oxidized transition metal, alkali metal salt or their mixture and matured at the room temperature for 2 to 3 days, and then filtered and dried at 70 to 110°C to make the multi-layered carbon nanoball to which the deodorizing material 17 is impregnated according to the present invention. As for the transition metal or the oxidized transition metal which may be impregnated to the shell, Copper (Cu), Iron (Fe), Manganese (Mn), Nickel (Ni), Cobalt (Co), Silver (Ag), Gold (Au), Vanadium (V), Ruthenium (Ru), Titanium (Ti), Chrome (Cr), Zinc (Zn), Palladium (Pd) or their oxide may be used. As for the alkali metal, sodium bromide (NaBr), sodium iodide (NaI), potassium bromide (KBr), potassium iodide (KI) and potassium iodate (KIO₃) may be used. An impregnated amount of the deodorizing material 17 may be controlled by changing the concentration of the deodorizing material solution or the infiltration time, and is preferably in the range of 0.01 ~ 30 wt% on the basis of the total weight of the ball shaped carbon structure for deodorization.

The ball shaped carbon structure impregnated by the deodorizing material according to the present, invention may be provided with one or more kinds of deodorizing materials among the above-mentioned metals. Thus, the deodorizing agent containing the ball shaped carbon structure impregnated by the deodorizing material according to the present invention may be prepared or composed in various ways depending on the kind of bad smell or its usage. For example, the deodorizing agent may contain the ball shaped carbon structure to which only one kind of deodorizing material is impregnated, or to which two different kinds of deodorizing materials are impregnated, or more than two kinds of deodorizing materials are impregnated.

The ball shaped carbon structure impregnated by the deodorizing material according to the present invention may be used for deodorizing and eliminating various odor materials such as methanethiol, methyl sulfide, dimethyl disulfide, hydrogen sulfide, ammonia, trimethylamine, styrene, acetaldehyde, nitric oxide, nitrous oxide, indoor bad smells generated in bathroom, kitchen or footwear cabinet in home, and smell of tobacco. Thus it also may give excellent effects in eliminating bad smells of refrigerator, air conditioner, air cleaner, automobile room, exhaust gas of cars as well as a human body.

In addition, the ball shaped carbon structure impregnated by the deodorizing materials according to the present invention may be uniformly dispersed and stuck to one having a shape of sheet, pack or pad, thus it may be applied to goods such as a diaper for the infant or the person suffered from the incontinence or a hygienic band for women, which use such matters.

### Embodiment 1 to 8

A spherical silica core is composed according to the well-known Stober method by using tetraethoxysilane as a silica precursor. At this time, 60mL of tetraethoxysilane is put into a homogeneous mixture solvent including 1000mL of ethanol, 80mL of water and 40mL of 28% concentration aqueous ammonia to form a spherical silica core of 200 to 300nm.

Then, 59mL of mixture in which octadecyltrimethoxysilane (C₁₈-TMS) and tetraethoxysilane are mixed at a molar ratio of 11.8, which is a surface active agent, is put into 1180mL of silica core and then reacted to grow the first shell on the surface of the silica core. And then, 59mL of mixture in which octadecyltrimethoxysilane and tetraethoxysilane are mixed at a molar ratio of 47.2 is additionally put therein and reacted for growing the second shell. Subsequently, the composed silica particle is filtered and then thermally treated at 550°C for 5 hours so that mesoporous pores having a certain size are formed in the place where the surface active agent is removed.

Then, divinylbenzene is sufficiently mixed with azobisisobutyronitrile (AIBN), which is a radical initiator, and then injected into the mesoporous pores of the silica particle, and then polymerized at 80°C for about 12 hours to make a silica structure containing polymer. In succession, the silica structure containing polymer is carbonized under the nitrogen atmosphere at 1,000°C to form a ball shaped carbon structure. Subsequently, the ball shaped carbon structure is put into hydrofluoric acid to remove inorganic structure of the ball shaped carbon structure, so the ball shaped carbon structure having a ball-shaped carbon structure including a hollow core and a porous multi-layered shell is obtained.

After that, in order to impregnate the deodorizing materials of the following Table 1, the ball shaped carbon structure made along with the above-mentioned method is dipped into 1N of the deodorizing material solution for about 50 hours, then filtered and dried at 70°C, so a ball impregnated by the deodorizing materials is obtained.

**Table 1**

| | The kind of impregnated metal |
|---|---|
| | (impregnated amount of metal, %) |
| Embodiment 1 | copper (1.3) + manganese (0.3) |
| Embodiment 2 | nickel (3.1) + iron (0.8) |
| Embodiment 3 | gold (0.8) + chrome (0.9) + palladium (0.8) |
| Embodiment 4 | copper (3.1) + iron (0.8) + zinc (0.8) |
| Embodiment 5 | potassium iodide (3.4) |
| Embodiment 6 | silver (4.2) |
| Embodiment 7 | cobalt (2.1) + potassium iodate (1.3) |
| Embodiment 8 | vanadium (2.1) + ruthenium (0.3) + titanium (0.6) |

In Table 1, Impregnated Amount (%) of Metal = Weight of Metal/Weight of ball shaped carbon structure × 100.

### Comparative Examples 1 and 2

Ball shaped carbon structure which mainly includes impregnated activated carbon as a deodorizing material, manufactured by S company and L company are selected as comparative examples 1 and 2.

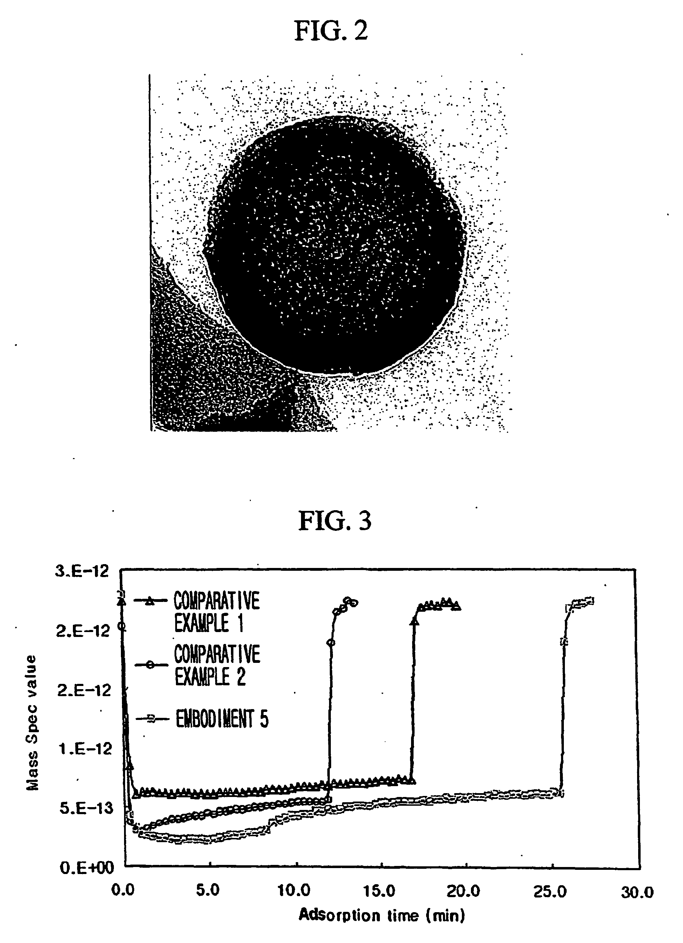

Deodorizing abilities against methanethiol of the ball shaped carbon structure according to the embodiment 5 and the comparative examples 1 and 2 are compared, and the comparison results are shown in FIG. 3. Methanethiol of 50 ppm is passed at a rate of 100 mL/min through a reactor containing the deodorizing materials, and its dynamic absorption behavior according to time is analyzed with a mass spectrometer. Referring to FIG. 3, it would be understood the ball shaped carbon structure impregnated by the deodorizing materials, which has a multi-layered shell structure according to the present invention, shows continuously excellent deodorizing effects rather than the comparative examples.

### INDUSTRIAL APPLICABILITY

As described above, the multi-layered ball shaped carbon structure impregnated by deodorizing materials according to the present invention adsorbs various kinds of malodor substances as well as shows good deodorizing ability. Thus, the ball shaped carbon structure of the present invention may show excellent deodorizing effect in capturing and resolving the malodor substances when it is used as a deodorizing agent in various odorizing daily necessaries, living spaces, industrial spots and other various stink-generating circumstances.

## Claims

1. A ball-shaped carbon structure (20) for deodorization comprising a porous carbon shell (13) having a spherical hollow core (15),
wherein at least one deodorizing material (17) selected from the group consisting of transition metal, oxidized transition metal and alkali metal salt is impregnated to the shell,
wherein the porous carbon shell (13) has a multi-layered structure in which at least two layers having different pore sizes are included, and
wherein an average diameter of pores formed in an outer layer is larger than an average diameter of pores formed in an inner layer or vice versa and wherein the spherical hollow core (15) has a diameter of 5 ~ 1,000nm, and the porous carbon shell (13) has a thickness of 10 ~ 500nm.

2. A ball shaped carbon structure (20) for deodorization according to claim 1,
wherein the transition metal is one selected from the group consisting of Copper (Cu), Iron (Fe), Manganese (Mn), Nickel (Ni), Cobalt (Co), Silver (Ag), Gold (Au), Vanadium (V), Ruthenium (Ru), Titanium (Ti), Chrome (Cr), Zinc (Zn) and Palladium (Pd), and
wherein the alkali metal salt is one selected from the group consisting of sodium bromide (NaBr), sodium iodide (NaI), potassium bromide (KBr), potassium iodide (KI) and potassium iodate (KIO₃).

3. A ball shaped carbon structure (20) for deodorization according to claim 1 or 2,
wherein an impregnated amount of the deodorizing material (17) is 0.01 ~ 30 wt% on the basis of the total weight of the ball shaped carbon structure (20) for deodorization.

## Patentansprüche

1. Eine ballförmige Kohlenstoffstruktur (20) für die Desodorierung, umfassend eine poröse Kohlenstoffumhüllung (13) mit einem sphärischen hohlen Kern (15),
wobei die Umhüllung mit mindestens einem desodorierenden Material (17), das aus der Gruppe ausgewählt ist, bestehend aus einem Übergangsmetall, einem oxidierten Übergangsmetall und einem Alkalimetallsalz imprägniert ist,
wobei die poröse Kohlenstoffumhüllung (13) eine mehrschichtige Struktur besitzt, in der zumindest zwei Schichten mit unterschiedlichen Porengrößen umfasst sind, und
wobei ein mittlerer Durchmesser der Poren, die in einer äußeren Schicht gebildet werden, größer ist, als ein mittlerer Durchmesser von Poren, die in einer inneren Schicht gebildet werden oder umgekehrt, und
wobei der sphärische hohle Kern (15) einen Durchmesser von 5 ~ 1000 nm und die poröse Kohlenstoffumhüllung (13) eine Dicke von 10 -500 nm aufweist.

2. Eine ballförmige Kohlenstoffstruktur (20) für die Desodorierung gemäß Anspruch 1,
wobei das Übergangsmetall ausgewählt ist aus der Gruppe bestehend aus Kupfer (Cu), Eisen (Fe), Mangan (Mn), Nickel (Ni), Kobalt (Co), Silber (Ag), Gold (Au), Vanadium (V), Ruthenium (Ru), Titan (Ti), Chrom (Cr), Zink (Zn) und Palladium (Pd) und
wobei das alkalische Metallsalz ausgewählt ist aus der Gruppe bestehend aus Natriumbromid (NaBr), Natriumjodit (NaI), Kaliumbromid (KBr), Kaliumiodit (KI) und Kaliumjodat (KIO₃).

3. Eine ballförmige Kohlenstoffstruktur (20) für die Desodorierung gemäß Anspruch 1 oder 2,
wobei eine imprägnierte Menge des desodorierenden Materials (17) 0,01 - 30 Gew.-% auf der Basis des Gesamtgewichts der ballförmigen Kohlenstoffstruktur (20) für die Desodorierung beträgt.

## Revendications

1. Structure de carbone ronde (20) pour désodorisation comprenant une coque de carbone poreuse (13) ayant un noyau' creux sphérique (15),
dans laquelle au moins un matériau de désodorisation (17) choisi dans le groupe constitué par un métal de transition, un métal de transition oxydé et un sel de métal alcalin est imprégné sur la coque,
dans laquelle la coque de carbone poreuse (13) a une structure multicouche incluant au moins deux couches ayant des tailles de pore différentes, et
dans laquelle un diamètre moyen de pores formés dans une couche externe est supérieur à un diamètre moyen de pores formés dans une couche interne ou vice versa et
dans laquelle le coeur creux sphérique (15) a un diamètre de 5 à 1 000 nm, et la coque de carbone poreuse (13) a une épaisseur de 10 à 500 nm.

2. Structure de carbone ronde (20) pour désodorisation selon la revendication 1,
dans laquelle le métal de transition est choisi dans le groupe constitué par le cuivre (Cu), le fer (Fe), le manganèse (Mn), le nickel (Ni), le cobalt (Co), l'argent (Ag), l'or (Au), le vanadium (V), le ruthénium (Ru), le titane (Ti), le chrome (Cr), le zinc (Zn) et le palladium (Pd), et
dans laquelle le sel de métal alcalin est choisi dans le groupe constitué par le bromure de sodium (NaBr), l'iodure de sodium (NaI), le bromure de potassium (KBr), l'iodure de potassium (KI) et l'iodate de potassium (KIO₃).

3. Structure de carbone ronde (20) pour désodorisation selon la revendication 1 ou 2,
dans laquelle une quantité imprégnée du matériau de désodorisation (17) est de 0,01 à 30 % en poids sur la base du poids total de la structure de carbone ronde (20) pour désodorisation.
